# EUROPÄISCHE PATENTSCHRIFT

(11) **EP 1 201 749 B1**
(45) Veröffentlichungstag und Bekanntmachung des Hinweises auf die Patenterteilung: **15.08.2007**
(21) Anmeldenummer: 01117785.4
(22) Anmeldetag: 02.08.2001
(51) Int. Cl.: C12N 5/06

(54) **Verfahren zur Herstellung von Knorpelimplantaten mittels in vitro gezüchteter Chondrozyten**
Method for producing cartilage implants using chondrocytes grown in-vitro
Procédé pour produire des implants de cartillage en utilisant des chondrocytes cultivés in-vitro

(30) Priorität: 29.08.2000 DE 10042484
(43) Veröffentlichungstag der Anmeldung: 02.05.2002
(73) Patentinhaber: Biomet Deutschland GmbH, 14167 Berlin (DE)
(72) Erfinder: Jeschke, Brigitte, Dr., 65779 Kelkheim (DE); Meyer, Jörg, Dr., 63150 Heusenstamm (DE); Adamietz, Peter, Dr., 22145 Hamburg (DE); Meenen, Norbert, Dr., 22587 Hamburg (DE); Göpfert, Christiane, 22527 Hamburg (DE)
(74) Vertreter: Gross, Felix

(56) Entgegenhaltungen:
- GRUBER ET AL: "Untersuchungen zur in-vitro-Kultivierung von Humanchondrozyten bei Einsatz von autologem Humanserum als Mediumzusatz: Minimierung des möglichen Risikos einer Infektion mit Erregern von Prionen-Erkrankungen" LARYNGO-RHINO-OTOLOGIE, THIEME, STUTTGART, DE, Bd. 75, Nr. 2, Februar 1996 (1996-02), Seiten 105-108, XP008000698 ISSN: 0935-8943
- DOMM ET AL: "Die Redifferenzierung von dedifferenzierten Gelenkknorpelzellen in Alginatkultur: Einfluss von intermittierendem hydrostatischen Druck und niedrigem Sauerstoffpartialdruck" ORTHOPÄDE, Bd. 29, Februar 2000 (2000-02), Seiten 91-99, XP002191525
- MÖLLER ET AL: "TGFbeta-1-Gentransfer in Gelenkknorpelzellen" ORTHOPÄDE, Bd. 29, Februar 2000 (2000-02), Seiten 75-79, XP002191526
- SCHULZE ET AL: "Adulte humane Chondrozyten in Alginatkultur: Beibehaltung des Phänotyps für die weitere Anwendung in Transplantationsmodellen" ORTHOPÄDE, Bd. 29, Februar 2000 (2000-02), Seiten 100-106, XP002191527
- WATERLOW & SCHÜRCH (HERAUSGEBER): "Proceedings of an I/D/E/G/C Workshop held in London, January 15-18, 1993; Section: The cell biology of bone growth: 7. Regulation of growth plate chondrocytes and bone cells" CAUSES AND MECHANISMS OF LINEAR GROWTH RETARDATION, [Online] XP002191528 Gefunden im Internet: <URL:http://www.unu.edu/unupress/food2/UID 06E/uid06e00.htm> [gefunden am 2002-02-13]

## Beschreibung

Die Erfindung betrifft ein Verfahren zur Herstellung von humanen Knorpelimplantaten aus in vitro gezüchteten Chondrozyten.
Artikulärer Knorpel leidet unter einer sehr begrenzten Fähigkeit zur Reparatur von Gelenkflächenschäden. Ein tragfähiges Konzept zur chirurgischen Therapie von Gelenkflächenschäden auf der Basis von *in vitro* hergestelltem Knorpel setzt unter anderem voraus, dass es gelingt Knorpelimplantate aus autologen Zellen (Chondrozyten oder mesenchymale Stammzellen) zu gewinnen, die hinsichtlich ihrer biochemischen Zusammensetzung und biomechanischen Eigenschaften dem Original möglichst nahe kommen. Prinzipiell eignen sich sowohl Chondrozyten als auch mesenchymale Stammzellen zu diesem Zweck.

Da autologe Zellen nur in geringer Zahl in Form einer Biopsie zur Verfügung stehen, ergibt sich die Notwendigkeit einer effektiven *in vitro* Expansion. Dies ist eine besondere Herausforderung. Denn wie aus der Literatur bekannt, wird stets ein mit der Anzahl der Passagen fortschreitender Verlust des differenzierten Phänotyps beobachtet. Das bedeutet: Während sich bei Einsatz primärer Zellen oder von Zellen der ersten Passage die Synthese von Knorpel bereits durch Intensivierung der Zell-Zell-Kontakte fördern lässt, versagt diese Methode, wenn Zellen zum Zwecke der Vermehrung häufiger passagiert werden müssen. Auch gelang es bei humanen Zellen bisher nicht, die Abnahme des Chondrogenesepotentials während der Vermehrungsphase durch verschiedene Zusätze wie bestimmte Seren oder bestimmte Wachstumsfaktoren wie bFGF aufzufangen.
Bereits bekannt ist die Vermehrung humaner Knorpelzellen in einer Zellkulturschale/flasche in Gegenwart sowohl von Kälberserum als auch humanen Serum (Gruber, R. et al Untersuchungen zur in-vitro-Kultivierung von Humanchondrozyten bei Einsatz von autologem Humanserum als Mediumzusatz: Minimierung des möglichen Risikos einer Infektion mit Erregern von Prionen-Erkrankungen Laryngo-Rhino-Otol. 1996 75:105-108, wobei die (Zellen in dieser Monolayer-Kultivierung) dedifferenzieren, mit anschließender Re-Differenzierung der Zellen in Alginat- Gel. Anschließend werden die Zellen und die sie umgebende Matrix mit unterschiedlichen Methoden innerhalb des Alginats oder nach Herauslösen der Zellen aus dem Alginat analysiert (Bonaventure J. et al., Exp. Cell Res. 212 (1):97-104 (1994) Reexpression of cartilage-specific genes by dedifferentiated human articular chondrocytes cultures in alginate; Yaeger P. C. et al. Exp. Cell Res. 237:318-325 (1997) Synergistic Action of Transforming Growth Factor-β and Insulin-like Growth Factor-I induces Expression of Type II Collagen and Aggrecan Genes in adult human articular Chondrocytes).

Der Einfluss des Sauerstoffpartialdruckes und des hydrostatischen Druckes während der Alginatkultivierung ist bereits untersucht worden (Domm, C. et al., Die Redifferenzierung von dedifferenzierten Gelenkknorpelzellen in Alginatkultur, Orthopädie, 2000, 29:911-99) ebenso wie die Bedeutung der Zugabe von Wachstumsfaktoren während des Re-differenzierungsprozesses (Möller, H.D. et al., TGFβ-1-Gentransfer in Gelenkknorpelzellen, Orthopädie, 2000, 29: 75-79; Schulze, M. et al. Adulte humane Chondrozyten in Alginatkultur, Orthopädie, 2000, 29:100-106)

Aufgabe der vorliegenden Erfindung ist es nun ein Verfahren zur Züchtung eines weitgehend differenzierten Knorpelgewebes (nicht einzelne Zellen) von ausreichender Größe anzugeben, welche die vorher genannten Nachteile aus dem Stand der Technik vermeidet, so dass das Knorpeigewebe in Gelenkknorpeldefekte "press-fit" (d.h. mit rotationsstabilem Sitz) implantiert werden kann.

Diese Aufgabe ist durch ein Verfahren zum Herstellen eines humanen Knorpelimplantates aus *in vitro* gezüchteten Chondrozyten mit den im Patentanspruch 1 angegebenen kennzeichnenden Merkmalen gelöst.

Das im Patentanspruch 1 dargestellte Verfahren läßt sich nicht nur auf humane Chondrozyten anwenden. Es eignet sich ebenfalls für Schweine- Chondrozyten, wie in einem Tiermodell gezeigt werden konnte. Denkbar ist auch ein Einsatz mit Chondrozyten anderer Spezies (z. B. Kamel, Dromedar, Pferd, Hund, Katze).

Das erfindungsgemäße Verfahren nutzt dabei, wie oben schon erwähnt, das herkömmliche Verfahren, Chondrozyten in ein Alginat-Gel einzuschließen, um sie zu re-differenzieren. Entscheidend für den Erfolg sind jedoch die modifizierten Kulturbedingungen, wobei die Verwendung eines humanen Serums (anstatt eines fötalen Kälber-Serums) als bis zu 20 Vol.% Medium-Zusatz, der Zusatz chondrogener Wachstumsfaktoren (IGF-I und TGF-β) und die Reduzierung des Sauerstoffpartialdruckes auf unter 10 Vol.% essentiell sind. Nur diese Bedingungen, über einen Zeitraum von mehreren Wochen appliziert, führen zu Chrondrozyten, die sich anschließend zur eigentlichen Knorpelbildung aggregieren lassen. Alternativ kann anstelle von IGF-I auch IGF-II verwendet werden. Die Zugabe des Cytokins Interleukin 4 (IL-4) stimuliert die Knorpelbildung zusätzlich.

Bei *in vitro* expandierten Chondrozyten wurde der Einschluss in Alginat-Gel bisher als Methode beschrieben, die sich eignet, das prinzipielle Chondrogenesepotential nachzuweisen (Yaeger P. C. et al. Exp. Cell Res. 237:318-325 (1997) Synergistic Action of Transforming Growth Factor-β and Insulin-like Growth Factor-I induces Expression of Type II Collagen and Aggrecan Genes in adult human articular Chondrocytes). Beim erfindungsgemäßen Verfahren eignet sich diese Methode (ohne den Einsatz des humanen Serums und die Reduzierung des Sauerstoffpartialdrucks) jedoch nicht für den präparativen Einsatz. Die resultierenden Chondron-artigen Gebilde (im folgenden als "Chondrone" bezeichnet), die sich durch vorsichtiges Auflösen des Gels durch Chelatbildner wie Zitrat gewinnen lassen, weisen einen hohen Kollagen Typ I- Gehalt auf und zeigen nicht die Neigung, bei Aggregation ein Knorpelgewebe zu bilden, wenn eine der aufgeführten erfindungswesentlichen Bedingungen nicht eingehalten wird.

Die besondere Bedeutung des Einsatzes von "Chondronen" für die Bildung von hyalinem Knorpel mit minimalem Gehalt an Kollagen Typ I ist auch an einem Tiermodell nachgewiesen worden. Nur, wenn in Alginat-Gel eingeschlossenen *in vitro* expandierten Chondrozyten des Schweins für 8 bis 12 Tage Gelegenheit gegeben wird, eine Chondron-artige extrazelluläre Matrix zu bilden, sind die Voraussetzungen für die anschließende Bildung hyalinen Knorpels mit minimalem Kollagen Typ I-Gehalt gegeben. Während die Re-Differenzierung bei humanen Chondrozyten eine mehrwöchige Kultivierung unter den oben genannten Bedingungen erfordert, reichen bei Chondrozyten des Schweins 8-12 Tage bei normalem (21%) Sauerstoffpartialdruck und der Zusatz von chondrogenen Wachstumsfaktoren, um hyalinen Knorpel mit hohem Kollagen Typ II/ Kollagen Typ I-Quotienten zu bilden.

Ferner bevorzugt ist ein Verfahren gemäß Anspruch 2, wobei die isolierten Chondrozyten aus dem Alginat nach dem Zentrifugieren auf einer ebenen Fläche Knorpel mit flächiger Gestalt bilden. Die Chondrozyten werden in ein Zentrifugenröhrchen mit planem Boden zentrifugiert. Dabei bilden sich eher flächige Knorpel-Aggregate wie sie für die Implantation benötigt werden. Die flächige Form der Implantate bietet wegen der kurzen Diffusionsstrecken zudem eher einen Vorteil für Versorgung / Entsorgung, während der *in vitro* Kultivierung.
Bevorzugt ist weiterhin ein Verfahren gemäß Anspruch 3, wobei als chondrogene Wachstumsfaktoren IGF-I und und TGF-β im Verhältnis 5 10 : 1 (w : w) zugesetzt werden. Ebenso bevorzugt ist die Addition von 0.1 - 3 ng/ml Interleukin 4 zur weiteren Förderung der Chondrogenese.

Ferner bevorzugt ist ein Verfahren, wobei der Sauerstoffpartialdruck auf unter 10% reduziert wird. Besonders bevorzugt ist ein reduzierter Partialdruck von 5%. Wie wichtig der Einfluss von reduziertem Sauerstoffpartialdruck auf das Verhältnis von Kollagen Typ I zu Typ II zur Differenzierung humaner Chondrozyten ist, kann man der im Anhang befindlichen Abb. 1 entnehmen.

Außerdem bevorzugt ist ein Verfahren, in dem zur Herstellung der Aggregat-Kultur bis zu 20 Vol.% humanes Serum als Medium-Zusatz verwendet werden. Besonders bevorzugt ist ein Medium-Zusatz von 10 Vol.% humanes Serum.

Bevorzugt ist auch ein Verfahren gemäß Anspruch 6, wobei die Chondrozyten vorteilhafterweise bis zur 12. Passage, besonders bevorzugt bis zur 7. Passage, in Monolayer-Kultur gehalten werden können.

Ferner bevorzugt ist ein Verfahren gemäß Anspruch 8, wobei ein hoher Kollagen Typ II / Typ I- Quotient resultiert. Ein hoher Kollagen Typ II / Typ I- Quotient ist ein Indiz dafür, dass Kollagen Typ I wie erwünscht vorzugsweise in der dünnen Außenschicht vorkommt. Genaue quantitative Aussagen lassen sich aus immunologischen Analysen ableiten, sind jedoch von der absoluten Größe der Proben abhängig. Aus dem Stand der Technik werden für die präferentiell aus Kollagen Typ I bestehende Oberflächenschicht Werte von 10-20% angegeben (T. Minas & S. Nehrer Orthopedics 20 (6): 525-538 (1997) Current concepts in treatment of articular cartilage defects).

Die vor- und nachstehend aufgeführten Abkürzungen haben folgende Bedeutung:

| | |
|---|---|
| PBS | Phosphatgepufferte Kochsalzlösung (Phosphate buffered Saline) |
| DMEM | Dulbecco's Modifiziertes Eagle Medium, high Glucose |
| DMEM/Ham's F12 | ......./Nutrient Mix F12 (1:1) |
| bFGF | basic Fibroblast Growth Factor |
| EGF | Epidermal Growth Factor |
| HEPES | N-2-Hydroxyethylpiperazin-N'-2-Ethan- Sulfonsäure |
| IGF bzw. \|GF-\| | Insulin like Growth Factor 1 |
| TGF-β | Transforming Growth Factor β |
| lL-4 | Interleukin 4 |
| w : w | weight by weight |

Die Erfindung wird nachfolgend anhand von zwei Ausführungsbeispielen näher beschrieben und erläutert.

### Ausführungsbeispiel 1:

### Isolierung der Chondrozyten:

Gelenkknorpel aus dem Radiuskopf eines 60 jährigen Patientin wurde abgeschält, von allen Blut- und Geweberesten befreit und in einer Petrischale mit Skalpellen zerkleinert. Die Knorpelstücke wurden auf das Sieb einer Verdauungskammer mit Magnetrührer gegeben und mit 50 ml Hyaluronidase-Lsg. (25 mg Hyaluronidase in 50 ml PBS) für 25 min bei 37°C gerührt. Danach wurde das Gewebe mit 50 ml 0.25 Gew.% Trypsin/ EDTA inkubiert (45 min., 37°C, gerührt) und 5 Minuten mit 50 ml DMEM + 10 Vol.% FKS gewaschen. Das Herauslösen der Chondrozyten aus dem Gewebeverband erfolgte mit einer dreimaligen Kollagenase-Behandlung (Kollagenase 1a), bei der das Gewebe mit 50 ml Kollagenase-Lsg. (25 mg in 50 ml DMEM + 10 Vol.% FKS + 100 U/ml Penicillin + 100 µg/ml Streptomycin) unter Rühren bei 37°C inkubiert und die Zellen aus der durchgetropften Lösung abzentrifugiert (500 x g, 5 min) wurden. Das Gewebe wurde zuerst für 2 Stunden, später zweimal über Nacht mit Kollagenase verdaut. Anschließend wurden weitere Zellen durch Zugabe von DMEM + 10 Vol.% FKS aus dem Gewebe gespült und abzentrifugiert wie zuvor.

### Monolayer-Kultivierung:

Die isolierten Chondrozyten wurden in einer Dichte von 10⁴ Zellen/cm² in Zellkulturflaschen ausgesät und mit DMEM/Ham's F12 (1/1) + 10 Vol.% FKS + 10 ng/ml bFGF + 1 ng/ml EGF kultiviert. Ein Mediumwechsel erfolgte zweimal pro Woche, wobei die Zellen einmal pro Woche mit 0.25 Gew.% Trypsin/ EDTA passagiert wurden. Die Chondrozyten wurden bis zur 7. Passage in Monolayer-Kultur gehalten.

### Alginat-Kultur:

Nach einem Waschschritt in Puffer NH (0.15 M NaCI + 25 mM Hepes, pH 7.4) wurden die Zellen in Puffer NH + 1.2 Gew.% Kaliumalginat aufgenommen (Dichte 1 Mio. Zellen/ml). Jeweils 1 ml der Zellsuspension wurde in eine Vertiefung einer 12-er-Platte (gefüllt mit 3 ml 0.1 M CaCl₂/25 mM Hepes) getropft. Die Verkapselung der Zellen im Alginat erfolgte sofort. Die Alginat-Kügelchen wurden nach 15 Minuten zweimal in Puffer NH gewaschen.
Anschließend wurden die im Alginat verkapselten Zellen in DMEM + 10 Vol.% humanem Serum + 50 µg/ml Ascorbinsäure + 100 ng/ml IGF + 10 ng/ml TGF-β aufgenommen und mit dreimal wöchentlichem Mediumwechsel für 3 Wochen bei 37°C, 5% CO₂, 95% relativer Luftfeuchtigkeit im Brutschrank kultiviert. Dabei wurden verschiedene humane Seren als auch FKS getestet. O₂-Partialdrücke von 5% und 21% wurden miteinander verglichen (siehe Abb. 1).

Zur Isolierung der Zellen aus dem Alginat wurden diese zunächst zweimal mit Puffer NH gewaschen, bevor durch Zugabe von Puffer NH + 55 mM Natriumcitrat das Alginat unter Taumeln für 15 Minuten aufgelöst wurde.

### Aggregat-Kultur:

Die isolierten Zellen wurden erneut mit Puffer NH gewaschen und auf den mit Kunstharz ausgegossenen Boden eines 15 ml Greiner-Röhrchen zentrifugiert (50 x g, 10 Min). Nach 2 Tagen Kultivierung mit DMEM + 10 Vol.% humanem Serum + 50 µg/ml Ascorbinsäure + 100 ng/ml IGF + 10 ng/ml TGF-β wurden das Aggregat umgebettet in eine mit Agarose beschichtete Vertiefung einer 12-er Platte. Die Kultivierung der aggregierten Zellen erfolgte in DMEM + 10 Vol.% humanem Serum + 100 ng/ml IGF + 10 ng/ml TGF-β + Penicillin/ Streptomycin für 2-3 Wochen unter den gleichen Bedingungen wie in der Alginat-Kultur, jedoch bei 21% O₂-Partialdruck.
Das Verfahren war mit verschiedenen humanen Seren, nicht aber mit FKS erfolgreich.

### Ausführungsbeispiel 2:

Isolierung der Chrondozyten und Monolayer-Kultivierung erfolgte wie in Beispiel 1 beschrieben.
Auch die Herstellung der Alginat- und Aggregat-Kultur erfolgte wie in Beispiel 1 beschrieben. Zur weiteren Stimulierung der Chondrogenese wurden jedoch 2 ng/ml des Cytokins IL-4 während der Alginat- und Aggregat-Kultur zugefügt.

### Beschreibung der Abbildung 1:

FCS = FKS; HS1 - HS4 = verschiedene humane Seren
CII = Kollagen II (Kontrolle)
Cl = Kollagen I (Kontrolle)

Bei 21% O₂ sind die Banden dicker als bei 5% O₂. Bei 21% O₂ entsteht aber auch mehr Kollagen Typ 1 → Der Quotient Kollagen Typ II/Typ I ist ungünstig.
Bei 5% O₂ wird nur wenig Kollagen Typ I bei der Verwendung von humanem Serum gebildet. Wird dagegen FKS benutzt, so verschlechtert sich das Verhältnis Kollagen Typ II/ Typ I deutlich.

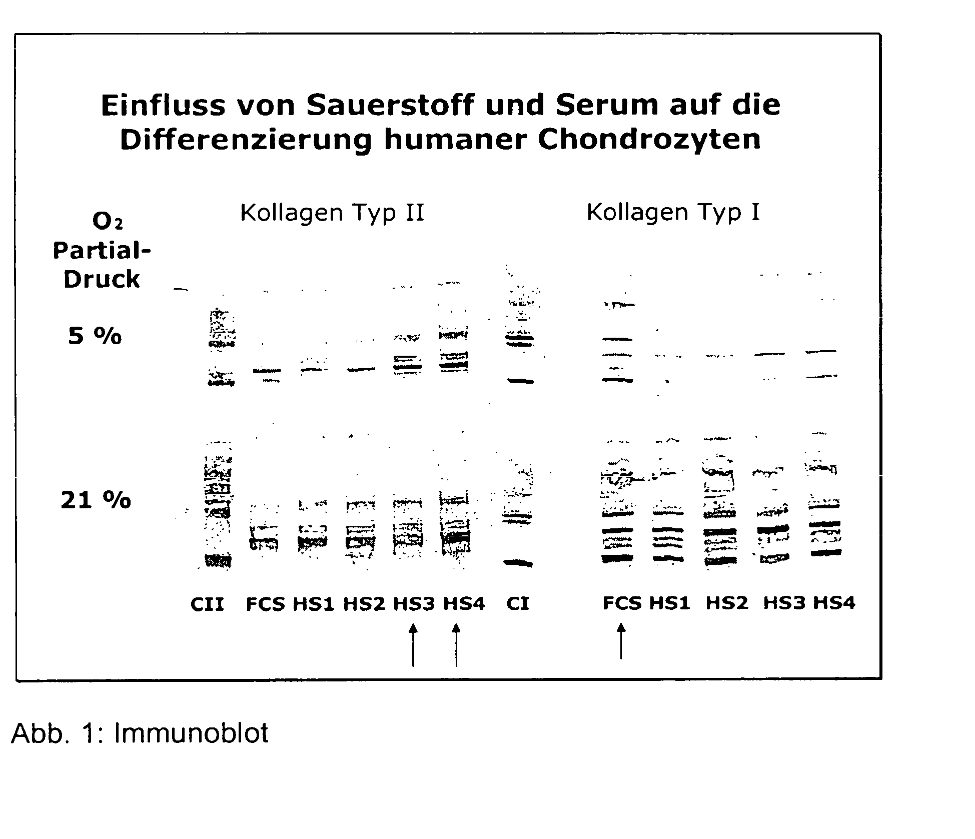

## Patentansprüche

1. Verfahren zum Herstellen eines humanen Knorpelimplantates aus in vitro gezüchteten Chondrozyten, **gekennzeichnet durch** folgende Merkmale:
aus patienteneigenen Knorpeln werden auf herkömmliche Weise **durch** Zugabe von verschiedenen Enzymlösungen Chondrozyten isoliert;
die isolierten Chondrozyten werden in Zellkulturflaschen ausgesät und in einer Nährlösung, versetzt mit humanem und/oder Kälberserum und Wachstumsfaktoren, kultiviert;
die gereinigten Chondrozyten werden in einer Alginat-haltigen Pufferlösung aufgenommen, wobei sofortige Verkapselung erfolgt, anschließend mehrere Wochen bei 34 - 39° C unter reduziertem Sauerstoff-Partialdruck kultiviert und die Zellen aus dem Alginat **durch** Zugabe von Citrat-Pufferlösungen isoliert;
die isolierten Chondrozyten werden zentrifugiert, einige Tage mit chondro-genen Wachstumsfaktoren, humanem Serum und weitere Zusätze enthaltender Nährlösung kultiviert, das Aggregat in eine mit Agarose beschichtete Vertiefung einer Platte umgebettet und die aggregierten Zellen unter den gleichen Bedingungen wie in der Alginat-Phase kultiviert, jedoch bei 21 % O₂- Partialdruck.

2. Verfahren zum Herstellung eines Knorpelimplantats gemäß Anspruch 1, **dadurch gekennzeichnet, dass** die isolierten Chondrozyten, die in Zellkulturflaschen ausgesät werden, in einer Nährlösung, versetzt mit humanem und/oder Kälberserum und Wachstumsfaktoren sowie Cytokinen, kultiviert werden.

3. Verfahren nach Anspruch 1 und/oder 2, **dadurch gekennzeichnet, dass** die isolierten Chondrozyten aus dem Alginat nach dem Zentrifugieren auf einer ebenen Fläche Knorpel mit flächiger Gestalt bilden.

4. Verfahren nach einem der Ansprüche 1 bis 3, **dadurch gekennzeichnet, dass** als chondrogene Wachstumsfaktoren IGF-I und TGF-β im Verhältnis 5 - 10 : 1 zugesetzt werden.

5. Verfahren nach Anspruch 2, **dadurch gekennzeichnet, dass** als Cytokin IL-4 in der Konzentration von 0.1 bis 3 ng/ml zugesetzt wird.

6. Verfahren nach einem der Ansprüche 1 bis 5, **dadurch gekennzeichnet, dass** der Sauerstoffpartialdruck auf unter 10 % reduziert wird.

7. Verfahren nach einem der Ansprüche 1 bis 6, **dadurch gekennzeichnet, dass** zur Herstellung der Aggregat-Kultur bis zu 20 Vol.% humanes Serum als Medium-Zusatz verwendet werden.

8. Verfahren nach einem der Ansprüche 1 bis 7, **dadurch gekennzeichnet, dass** die Chondrozyten bis zur 12. Passage in Monolayer-Kultur gehalten werden.

9. Verfahren nach einem der Ansprüche 1 bis 8, **dadurch gekennzeichnet, dass** die Chondrozyten bis zur 7. Passage in Monolayer-Kultur gehalten werden.

10. Verfahren nach einem der Ansprüche 1 bis 9, **dadurch gekennzeichnet, dass** ein hoher Kollagen Typ II / Kollagen Typ I-Quotient resultiert.

## Claims

1. Process for producing a human cartilage implant from chondrocytes grown in vitro, **characterized by** the following features:
chondrocytes are isolated from a patient's own cartilage in a conventional manner by addition of various enzyme solutions;
the isolated chondrocytes are seeded in cell culture vessels and cultivated in a nutrient solution admixed with human and/or calf serum and growth factors;
the purified chondrocytes are taken up in an alginate-containing buffer solution and immediate encapsulation ensues, then cultivated for several weeks at 34-39°C under reduced oxygen partial pressure and the cells are isolated from the alginate by addition of citrate buffer solutions;
the isolated chondrocytes are centrifuged, cultivated for some days with chondrogenic growth factors, human serum and nutrient solution containing further additives, the aggregate is re-embedded into an agarose-coated depression in a plate and the aggregated cells are cultivated under the same conditions as in the alginate phase but at 21% O₂ partial pressure.

2. Process for producing a cartilage implant according to Claim 1, **characterized in that** the isolated chondrocytes which are seeded in cell culture vessels are cultivated in a nutrient solution admixed with human and/or calf serum and growth factors and also cytokines.

3. Process according to Claim 1 and/or 2, **characterized in that** the isolated chondrocytes from the alginate form, after centrifuging, cartilage having a sheetlike shape on a planar surface.

4. Process according to any one of the Claims 1 to 3, **characterized in that** IGF-1 and TGF-β are used as chondrogenic growth factors in a ratio of 5-10:1.

5. Process according to Claim 2, **characterized in that** the cytokine added is IL-4 in a concentration of 0.1 to 3 ng/ml.

6. Process according to any one of the Claims 1 to 5, **characterized in that** the oxygen partial pressure is reduced to below 10%.

7. Process according to any one of the Claims 1 to 6, **characterized in that** up to 20% by volume of human serum is used as addition to the medium to produce the aggregate culture.

8. Process according to any one of the Claims 1 to 7, **characterized in that** the chondrocytes are maintained in monolayer culture up to the 12th pass.

9. Process according to any one of the Claims 1 to 8, **characterized in that** the chondrocytes are maintained in monolayer culture up to the 7th pass.

10. Process according to any one of the Claims 1 to 9, **characterized in that** a high collagen type II/collagen type I ratio results.

## Revendications

1. Procédé pour la production d'un implant de cartilage humain à partir de chondrocytes cultivés in vitro, **caractérisé par** les particularités suivantes:
on isole de façon classique des chondrocytes à partir de cartilages du patient, par addition de diverses solutions d'enzymes ;
on utilise les chondrocytes isolés pour ensemencer des flacons de culture cellulaire et on les cultive dans une solution nutritive additionnée de sérum humain et/ou de veau et de facteurs de croissance ;
on reprend les chondrocytes purifiés dans une solution de tampon contenant un alginate, de sorte qu'il s'effectue une encapsulation immédiate, puis on les cultive pendant plusieurs semaines à 34-39°C sous une pression partielle réduite d'oxygène et on isole les cellules de l'alginate par addition de solutions de tampon citrate ;
on centrifuge les chondrocytes isolés, on les cultive pendant quelques jours avec une solution nutritive contenant des facteurs de croissance chondrogènes, du sérum humain et d'autres additifs, on transfère l'agrégat dans un puits, tapissé d'agarose, d'une plaque, et on cultive les cellules agrégées dans les mêmes conditions que dans la phase d'alginate, mais sous une pression partielle d'O₂ de 21 %.

2. Procédé pour la production d'un implant de cartilage selon la revendication 1, **caractérisé en ce que** les chondrocytes isolés qui sont utilisés pour ensemencer les flacons de culture cellulaire sont cultivés dans une solution nutritive additionnée de sérum humain et/ou de veau et de facteurs de croissance ainsi que de cytokines.

3. Procédé selon la revendication 1 et/ou la revendication 2, **caractérisé en ce que** les chondrocytes isolés provenant de l'alginate forment après la centrifugation du cartilage de forme plate sur une surface plane.

4. Procédé selon l'une quelconque des revendications 1 à 3, **caractérisé en ce qu'**on ajoute en tant que facteurs de croissance chondrogènes de l'IGF - I et du TGF-β en un rapport allant de 5:1 à 10:1.

5. Procédé selon la revendication 2, **caractérisé en ce qu'**on ajoute en tant que cytokine de l'IL-4 à une concentration de 0,1 à 3 ng/ml.

6. Procédé selon l'une quelconque des revendications 1 à 5, **caractérisé en ce que** la pression partielle d'oxygène est réduite à moins de 10 %.

7. Procédé selon l'une quelconque des revendications 1 à 6, **caractérisé en ce que** dans la préparation de la culture d'agrégat on utilise jusqu'à 20 % en volume de sérum humain en tant qu'additif au milieu.

8. Procédé selon l'une quelconque des revendications 1 à 7, **caractérisé en ce que** les chondrocytes sont maintenus en culture monocouche jusqu'au 12^{e} transfert.

9. Procédé selon l'une quelconque des revendications 1 à 8, **caractérisé en ce que** les chondrocytes sont maintenus en culture monocouche jusqu'au 7^{e} transfert.

10. Procédé selon l'une quelconque des revendications 1 à 9, **caractérisé en ce qu'**il en résulte un quotient collagène de type II/collagène de type I élevé.
